(19) **Europäisches Patentamt**

**European Patent Office**

**Office européen des brevets**

(11) **EP 1 173 620 B1**

(12) **EUROPEAN PATENT SPECIFICATION**

(45) Date of publication and mention
of the grant of the patent:
**08.11.2006 Bulletin 2006/45**

(21) Application number: **00928450.6**

(22) Date of filing: **26.04.2000**

(51) Int Cl.:
*C12Q 1/68* (2006.01)     *G01N 33/543* (2006.01)

(86) International application number:
**PCT/US2000/011282**

(87) International publication number:
**WO 2000/065097 (02.11.2000 Gazette 2000/44)**

(54) **BIOCHIP AND METHOD FOR MAKING SAME**

BIOCHIP SOWIE VERFAHREN ZU DESSEN HERSTELLUNG

BIOPUCES ET PROCEDE DE FABRICATION

(84) Designated Contracting States:
**AT BE CH CY DE DK ES FI FR GB GR IE IT LI LU MC NL PT SE**

(30) Priority: **26.04.1999 US 299831**

(43) Date of publication of application:
**23.01.2002 Bulletin 2002/04**

(73) Proprietor: **Biocept, Inc.**
**San Diego, CA 92121 (US)**

(72) Inventors:
• **HAHN, Soonkap**
  **San Clemente, CA 92672 (US)**
• **FAGNANI, Roberto**
  **La Jolla, CA 92037 (US)**
• **TSINBERG, Pavel**
  **San Diego, CA 92122 (US)**

(74) Representative: **HOFFMANN EITLE**
**Patent- und Rechtsanwälte**
**Arabellastrasse 4**
**81925 München (DE)**

(56) References cited:
**EP-A- 0 420 053          EP-A- 0 453 621**
**EP-A- 0 481 740          WO-A-94/09125**
**US-A- 4 098 645          US-A- 5 563 056**

EP 1 173 620 B1

**Description**

The present invention relates to new methods of making biochips and the biochips resulting therefrom. In particular, the new method described herein provides for rapid, simple and cost effective construction of biochips by employing isocyanate-functional hydrogels to immobilize biomolecular probes on the substrate. In particular, both organic-solvent-soluble biomolecules, like peptide nucleic acids (PNAs), and water-soluble biomolecules, like DNA, RNA and other oligonucleotides, are easily and efficiently bound to a hydrophilic polymer either before or during polymerization thereof. In addition to the improved method of making the biochips described herein, the biochips themselves have improved characteristics including superior stability providing a much improved shelf-life and greater flexibility in use. Such biochips are useful for gene discovery, gene characterization, functional gene studies, screening for biological activity and related studies.

**BACKGROUND OF THE INVENTION**

Agents that selectively bind to DNA, RNA or analogs, such as peptide nucleic acids (PNAs) are of significant interest to molecular biology and medicinal chemistry as they may be developed into gene-targeted drugs for diagnostic and therapeutic applications and may be used as tools for sequence-specific modification of DNA. Additionally, such reagents may be used as tools for determining gene sequences and for functional gene analyses.

Until recently, the processes of gene discovery, characterization and functional gene analysis have been difficult and costly and have required tremendous amounts of time. However, within about the last ten years, methods of isolating arrays of biomolecules on various supports, referred to as biochips, have been developed and have been employed in DNA synthesis, sequencing, mutation studies, gene expression analysis and gene discovery. Generally the biochips are micromatrices (i.e., microarrays) of biomolecules bound to a substrate, either directly or through a linking group or, more recently, by way of a gel layer. Most biochips are designed to facilitate synthesis of biomolecules at known locations on a substrate. For example, one such biochip employs light and a series of photo-lithographic masks to activate specific sites on a substrate, such as glass, in order to selectively bind nucleic acids thereto and, subsequently, to attach additional nucleic acids to form known oligonucleotides at the desired locations. This process of using light and photolithographic masks to activate specific sites on a substrate is similar to the processes used in production of the microelectronic semiconductor chip.

Unfortunately, these first generation biochips are very expensive to produce, requiring large capital investments, process engineering and equipment. Furthermore, this synthesis method of forming oligonucleotides in a single layer on a substrate results in a low sensitivity biochip requiring an expensive laser confocal fluorescence microscope for adequate detection of DNA specifically hybridized to the chip. U.S. Patent No. 5,744,305, issued to Fodor, et al. (hereinafter, the '305 patent), provides an example of the use of photolabile protecting groups and photolithography to create arrays of materials attached to a substrate, describing a synthetic strategy for the creation of large scale chemical diversity wherein a substrate, such as glass, is derivatized, either directly or by addition of a linker molecule, to include an amino group blocked with a photolabile protecting group. Masking techniques are employed to permit selective deprotection of the photolabile groups within a specified, known location on the substrate. The deprotected region is then reacted with a "building block" molecule, for example an oligonucleotide, also containing a photolabile protecting group, such that the building block molecule is covalently bound to the active group at the surface of the substrate (or linker). This process is then repeated using the masks to direct synthesis of polymers, for example biomolecules, such as oligonucleotides or peptides, of specific sequences at predefined locations on the substrate.

The synthetic strategies described in the '305 patent contemplate providing from about 10 to about $10^8$ different sequences on a single substrate. Additionally, it is stated that the predefined regions on the substrate, wherein individual polymers are to be synthesized, are from about $10^{-10}$ cm$^2$ to about 1 cm$^2$. While the examples presented in the '305 patent primarily involve synthesis of peptides or nucleotides, it is stated that the same techniques may also be employed in the synthesis of other polymers. Similarly, various linker groups, preferably inactive or inert, for linking the synthesized polymer to the substrate are discussed in the '305 patent, as are various protecting groups for protecting an active site on the monomers which protecting groups may be selectively removed for directed synthesis of the polymer. Also discussed in some detail in the '305 patent is a binary masking technique utilized in one embodiment for directed synthesis of the array. Unfortunately, the strategies described in the '305 patent suffer from many of the same disadvantages as other prior art methods and apparatus. The arrays are expensive to manufacture and use, require multiple steps and lengthy incubation/washing times during manufacture and, significantly, permit synthesis in only a single layer.

In view of the low sensitivity of these first generation biochips, second generation biochips have been developed.

One example of a second generation biochip is described in U.S. Patent Nos. 5,736,257 and 5,847,019, issued to Conrad, et al. The '257 and 019 patents describe a process of synthesizing a biochip comprising providing a substrate, such as glass, having surface hydroxyl groups; reacting the substrate surface hydroxyl groups with silanes to bind a molecular layer of vinyl groups upon the substrate; placing an acrylamide compound on the molecular layer, which acrylamide compound can participate in a free radical polymerization reaction to make a polymerized network layer bound to the molecular layer; photo-activating the polymerized network layer to make a patterned photo-activated polymerized network; and placing upon the photo-activated polymerized network layer, for example by synthesis thereon, one or more similar or dissimilar biomolecules to bind to the patterned photo-activated polymerized network layer.

The biochips disclosed in the '257 and '019 patents are somewhat similar to the first generation biochips of Fodor, et al. in that they employ photolithographic techniques to direct binding (or synthesis) of biomolecules to an array. However, in contrast to the first generation biochips, the biochips of the '257 and '019 patents employ a polyacrylamide network on top of a molecular layer of vinyl groups, thereby giving a third dimension to the gel cells. Still, as will be readily appreciated by those of skill in the art, production of biochips in accordance with the disclosures of the '257 and '019 patents is not only expensive but also time-consuming.

U.S. Patent No. 5,552,270, issued to Khrapko, et al., describes a method of sequencing DNA which utilizes a second generation biochip comprising a solid support and a matrix that includes an array of oligonucleotides of desired lengths. The matrix is attached to the support by means of a gel layer having a thickness of 30 $\mu$m or less; a gel layer is described in the form of a set of spaced apart square "dots". In contrast to the single layer format described in the '305 patent, the gel layer of the '270 patent provides for a three-dimensional attachment of oligonucleotides to the substrate at a capacity therefore exceeding the capacity of the mono-molecular layer of the first generation biochips. This second generation biochip polymerizes a polyacrylamide gel between two glass slides spaced about 30 $\mu$m apart. One slide is removed, and the gel-coated lower slide is dried. Portions of the gel are then mechanically removed to leave the spaced dots. In alternative embodiments which also employ polyacrylamide gel matrices as described in U.S. Patent Nos. 5,741,700, 5,770,721 and 5,756,050, issued to Ershov, et al., gel parts are removed from the slide using a laser.

Although the polyacrylamide gel matrices described in the Ershov, et al. patents and the '270 Khrapko, et al. patent are hydrogels having a water content, at equilibrium, of about 95% to 97%, and provide favorable diffuseability for target molecules such as DNA's, they have significant disadvantages. A significant disadvantage to such second generation biochips is their cost. The polyacrylamide-based biochip described by Ershov, et al., is based upon the polymerization of acrylamide monomers by a free radical initiation or ultraviolet radiation process; however, this polyacrylamide-based gel biochip is constructed in a multi-step process that is lengthy and labor-intensive. Production of such a biochip requires cumbersome multi-step processing including polymerization and binding to the surface of the glass substrate; mechanical or laser cutting to form micro-squares of gel matrix on the substrate; activation step using hydrazines; and finally reaction with the oligonucleotides. Due to the polymerization process of inherent polyacrylamide gels, these steps must be performed independently. Thus, the total time required to produce a single biochip by such methods is at least about 24 to 48 hours. Furthermore, after each step, thorough washings and/or other special cares must be taken before the next step is begun. For example, the,oligonucleotide derivatization step requires a long incubation period, such as twenty-four to forty-eight hours. WO-A-94/09125 discloses a matrix of an absorbent foam, such as polyurethane, used in a bioreactor, which contains bioactive molecules. The active material may be suspended throughout the matrix by in-situ co-polymerisation.

Still another significant disadvantage to such second generation biochips lies in the fact that the reaction of the oligonucleotides with the hydrazine groups forms unstable morpholine derivatives resulting in a very short shelf half-life for the biochip of approximately thirty-six hours at room temperature. Thus, there is a significant need in the industry for a simple, cost effective, rapid method for constructing a reliable multi-functional biochip having high sensitivity and a reasonably long shelf-life that may be used in gene discovery, gene characterization, functional gene analysis and related studies.

## SUMMARY OF THE INVENTION

The present invention provides a rapid, simple, cost effective method for construction of an improved biochip and an improved biochip resulting therefrom. For example, the method described herein allows biomolecular probes to be bound prior to or simultaneously with polymerization of the gel. Further provided herein are improved biochips having increased sensitivity, superior stability, both in use and with respect to shelf-life, and improved cost effectiveness.

Such biochips can be formed by dispensing optically transparent microdroplets of hydrogel prepolymer having biomolecular probes suitably, e.g. covalently, attached thereto onto a substrate. It may however be preferable to prepare isocyanate-functional hydrogel biochips having a biomolecule immobilized thereon, by providing an organic solvent solution of an isocyanate-functional hydrogel prepolymer; providing a buffered aqueous solution of the biomolecule to be immobilized thereon; mixing the two solutions to covalently bind the biomolecule to the hydrogel prepolymer while initiating polymerization; and then dispensing the polymerizing hydrogel prepolymer onto a solid substrate so that the polymerized hydrogel becomes suitably bound to such substrate. In certain preferred embodiments, the hydrogel has sufficient active isocyanate groups thereon to both participate in the covalent binding of biomolecular probes to the hydrogel prepolymer as well as participate in polymerization of the hydrogel.

In one aspect, a method of preparing a hydrogel biochip having a biomolecule immobilized thereon, the method comprising the steps of (a) providing an organic solvent solution of isocyanate-functional hydrogel prepolymer; (b) providing a solution of a biomolecule; (c) covalently binding the biomolecule to the hydrogel prepolymer; and (d) initiating polymerization of the hydrogel prepolymer, is provided.

In another aspect, a method of preparing a biochip having an organic solvent soluble biomolecule covalently bound thereto is provided, the method comprising (a) providing a solid substrate having a top surface; (b) providing a solution comprising the biomolecule in an aprotic organic solvent; (c) providing an isocyanate-functional hydrogel prepolymer in an aprotic organic solvent; (d) derivatizing the prepolymer with the biomolecule from step (b); (e) initiating polymerization of the derivatized hydrogel prepolymer of step (d) by adding a buffered aqueous solution thereto; and (f) microspotting the polymerizing hydrogel solution of step (e) onto the top surface of the substrate of step (a), resulting in attachment of the hydrogel and immobilized biomolecule to the substrate.

In yet another aspect, a biochip comprising (a) a solid substrate having a top surface; (b) a plurality of hydrogel cells covalently bound to the top surface of the substrate, wherein each hydrogel cell is formed of an isocyanate-functional polymer; and (c) a biomolecular probe covalently bound to and within at least one of the hydrogel cells is provided. Preferably, a different biomolecular probe will be bound within each hydrogel cell, and preferably the covalent binding is via the isocyanate groups.

In a further aspect, a biochip comprising (a) a solid substrate having a top surface; (b) a plurality of hydrogel cells covalently bound to the top surface of the substrate, wherein the hydrogel comprises polyethylene glycol, polypropylene glycol, or copolymers thereof; and (c) different biomolecular probes which are covalently bonded to and within different hydrogel cells is provided.

In a still further aspect, a biochip comprising (a) a solid substrate having a top surface; (b) a plurality of hydrogel cells covalently bound to the top surface of the substrate wherein the hydrogel comprises a polymer with urethane-urea linkages; and (c) different biomolecular probes which are covalently bound to and within different hydrogel cells.

In a yet further aspect, a hybridization assay is provided, comprising (a) providing a biochip which comprises a substrate having at least two hydrogel cells bound thereto, each cell having a thickness of at least about 20 $\mu$m and comprising a major portion of a polymer selected from the group consisting of polyethylene glycol, polypropylene glycol and copolymers thereof, wherein at least one hydrogel cell further includes a biomolecular probe covalently bound thereto; (b) contacting the biochip with an analyte solution, containing a target biomolecule, under hybridization conditions; (c) washing the hydrogel biochip under conditions that remove non-specifically bound and unbound target biomolecule; and (d) detecting the bound target biomolecule. ,

In an additional aspect, a hybridization assay is provided, comprising (a) providing an organic solvent solution of a hydrogel prepolymer having an active isocyanate content of less than about 1 meq/g; (b) providing a solution of a biomolecule; (c) covalently binding the biomolecule to at least a portion of the hydrogel prepolymer; (d) initiating polymerization of the hydrogel prepolymer; (e) dispensing the polymerizing hydrogel prepolymer onto a solid substrate such that said solution is covalently bound to the substrate at a thickness of at least about 20 $\mu$m, thereby resulting in a biochip; (f) washing the biochip from step (e) with washing buffer such that remaining active sites within the hydrogel are blocked; (g) contacting the washed biochip with an analyte solution, containing a target biomolecule, under hybridization conditions; (h) then washing the biochip from step (g) with a second washing buffer such that non-specifically bound and unbound target biomolecule is removed; and (i) detecting the target biomolecule bound to the hydrogel chip.

Preferably each microdroplet of hydrogel on a biochip contains a different biomolecular probe, thereby permitting the screening of large numbers of biomolecular probes as a part of a single hybridization assay. In one preferred embodiment, the biomolecular probes used in constructing the biochips are PNA probes which provide superior screening functions as compared to traditional DNA and/or RNA oligonucleotides. Alternatively, DNA, RNA or other oligonucleotide probes are used in conjunction with a polyurethane-based or other isocyanate-functional gel matrix.

## BRIEF DESCRIPTION OF FIGURES

FIG. 1 is a schematic illustrating the reaction of a hydrogel prepolymer with an organic-soluble biomolecule followed by polymerization of the hydrogel, as a part of a process embodying various features of the present invention.

FIG. 2 is a schematic illustrating the alternative reaction of a hydrogel prepolymer with a water-soluble biomolecule, such as DNA, during polymerization of the hydrogel.

## DETAILED DESCRIPTION OF THE PREFERRED EMBODIMENTS

In order to provide a three-dimensional gel matrix useful in producing a biochip, the polymer chosen to comprise the gel matrix should preferably have a number of desirable properties, such as: 1) adequate pore size and high water content to permit diffusion of molecules in and out of the matrix; 2) an ability to bind to the surface of a substrate, such as glass; 3) sufficient transparency, in its fully polymerized state, to reduce optical interference with fluorescent tags; 4) sufficient structural integrity, when fully polymerized, to withstand the forces encountered during use; and 5) adequate shelf life for normal research and clinical use. Furthermore, the selected gel should preferably be easy to produce and use.

Hydrogels are a class of polymers that meet these criteria. Hydrogels are hydrophilic network polymers which are glassy in the dehydrated state and swollen in the presence of water forming an elastic gel.

In contrast to the polyacrylamide gel systems of Ershov, et al. and Khrapko, et al., it has now been discovered that isocyanate-functional hydrogels lack most of the disadvantages of the polyacrylamide-based gels while possessing a number of important advantages over the prior art. Isocyanate-functional hydrogel polymers are well known and have been used extensively in the production of absorbent materials such as surgical dressings, diapers, bed pads, catamenials, and the like.

By isocyanate-functional hydrogels are meant organic polymers that are capped with isocyanate groups that function to carry out the desired polymerization and may also covalently bind the biomolecules of interest. For example, they may be polyurethanes formed by reaction between diisocyanates and polyether or polyester polyols.

The prepolymers used as a starting material for these isocyanate-functional hydrogels preferably provide hydrated polyurethane, polyurea-urethane and/or polyurea polymer gels. Polyurethane polymers are well known in the art. Hydrogel polymers have been prepared from various prepolymers and used for a wide variety of applications. Typically, hydrogels are formed by polymerizing a hydrophilic monomer in an aqueous solution under conditions such that the prepolymer becomes cross-linked, forming a three-dimensional polymeric network which gels the solution in concentrated form. Polyurethane hydrogels are formed by polymerization of isocyanate-end-capped prepolymers to create urea and urethane linkages.

The isocyanate-functional prepolymers are often prepared from relatively high molecular weight polyoxyalkylene diols or polyols which are reacted with difunctional or polyfunctional isocyanate compounds. The preferred prepolymers made from polyoxyalkylene diols or polyols that comprise homopolymers of ethylene oxide units or block or random copolymers containing mixtures of ethylene oxide units and propylene oxide or butylene oxide units. In the case of block or random copolymers, at least 75% of the units should be ethylene oxide units. The polyoxyalkylene diol or polyol molecular weight is preferably from 2,000 to 30,000, more preferably from 5,000 to 30,000. Suitable prepolymers may be prepared by reacting selected polyoxyalkylene diols or polyols with polyisocyanate at an isocyanate-to-hydroxyl ratio of about 1.2 to about 2.2 so that essentially all of the hydroxyl groups are capped with polyisocyanate. The isocyanate-functional.prepolymer preferably used in this invention contains active isocyanates in an amount of about 0.1 meq/g to about 1 meq/g, preferably about 0.2 meq/g to about 0.8 meq/g. In general, a low molecular weight prepolymer, e.g. less than 2000, should contain a relatively high isocyanate content (about 1 meq/g or higher). The polymerization rate of some of these prepolymers may be very difficult to control and may result in too fast polymerization to effectively microspot. Also, these prepolymers having such high isocyanate content may leave a relatively high content of free amines after polymerization, whose positive charges can increase non-specific binding with negatively charged target DNA samples, resulting in high background signals. Thus, high molecular weight prepolymers containing a relatively low isocyanate content are preferred for certain applications.

Such high molecular weight prepolymers are often prepared by either of two general methods, but others can also be used:

(1) High molecular weight polyol (triol or higher, molecular weight at least 2000) is reacted with polyisocyanate such as isophorone diisocyanate; and
(2) High molecular weight diol (molecular weight at least 2000) is reacted with polyisocyanate and a cross-linking agent such as glycerol, trimethylolpropane, trimethylolethane, triethanolamine or organic triamine.

Aromatic, aliphatic or cycloaliphatic polyisocyanates may be used. The high molecular weight aliphatic isocyanate-capped prepolymers typically gel to a hydrated polymer state in about 20 to 90 minutes, whereas prepolymers capped with aromatic polyisocyanates gel much more rapidly. Examples of suitable di- and polyfunctional isocyanates are as follows: toluene-2,4-diisocyanate, toluene-2,6-diisocyanate, isophorone diisocyanate, ethylene diisocyanate, ethylidene diisocyanate, propylene-1,2-diisocyanate, cyclobexylene-1,2-diisocyanate, cyclohexylene-1,4-diisocyanate, m-phenylene diisocyanate, 3,3"-diphenyl-4,4"-biphenylene diisocyanate, 1,6-hexamethylene diisocyanate, 1,4-tetramethylene diisocyanate, 1,10-decamethylene diisocyanate, cumene-2,4-diisocyanate, 1,5-naphthalene diisocyanate, methylene-dicyclohexyl diisocyanate, 1,4-cyclohexylene diisocyanate, p-tetramethyl xylylene diisocyanate, p-phenylene diisocyanate, 4-methoxy-1,3-phenylene diisocyanate, 4-chloro-1,3-phenylene diisocyanate, 4-bromo-1,3-phenylene diisocyanate, 4-ethoxyl-1,3-phenylene diisocyanate, 2,4-dimethyl-1,3-phenylene diisocyanate, 2,4-dimethyl-1,3-phenylene diisocyanate, 5,6-dimethyl-1,3-phenylene diisocyanate, 1,4-diisocyanatodiphenylether, 4,4'-diisocyanatodiphenylether, benzidine diisocyanate, 4,6-dimethyl-1,3-phenylene diisocyanate, 9,10-anthracene diisocyanate, 4,4'-diisocyanatodi-benzyl, 3,3'-dimethyl-4,4'-diisocyanatodiphenylmethane, 1,6-dimethyl-4,4'-diisocyanatodiphenyl, 2,4-diisocyanatostibene, 3,3'-dimethoxy-4,4'-diisocyanatodiphenyl, 1,4-antracenediisocyanate, 2,5-fluoronediisocyanate, 1,8-naphthalene diisocyanate, 2,6-diisocyanatobenzluran, 2,4,6-toluene triisocyanate, p,p',p"-triphenylmethane triisocyanate, trifunctional trimer (isocyanurate) of isophorone diisocyanate, trifunctional biuret of hexamethylene diisocyanate, trifunctional trimer (isocyanurate) of hexamethylene diisocyanate, polymeric 4,4'-diphenylmethane diisocyanate, xylylene diisocyanate and m-tetramethyl xylylene diisocyanate.

Capping of the selected diols or polyols with polyisocyanates to form prepolymers may be effected using stoichiometric amounts of reactants. The isocyanate-to-hydroxyl group ratio may vary as known in this art but should preferably be about 1 to about 3, and more preferably about 1.2 to about 2.2. The capping reaction may be carried out using any suitable method or procedure, such as at about 20° to about 150°C, under dry nitrogen, for about 2 hours to about 14 days, preferably in the absence of a catalyst. The preferred temperature is about 60° to 100°C. The reaction terminates when the isocyanate concentration approximates theoretical values.

Preferred prepolymers include polyethylene glycol end-capped with toluene diisocyanate, toluene diisocyanate and copolymer of ethylene oxide and propylene oxide optionally with trimethylolpropane, toluene diisocyanate-polyethylene glycol-trimethylopropane, methylene diisocyanate-methylene homopolymer, polymeric methylene diisocyanate-polyethylene glycol, isophorone diisocyanate and polymer of ethylene oxide-propylene oxide-trimethylolpropane, and toluene diisocyanate polyethylene glycol trilactate. Such prepolymers are available from Hampshire Chemical Corp. of Lexington, Mass. as HYPOL PreMA®, HYPOL® 2000, HYPOL® 3000, HYPOL® 4000 and HYPOL® 5000 and include copolymers of polyethylene oxide and a minor amount of polypropylene oxide.

All things considered, the main chain of the hydrogel polymer is preferably comprised of polyethylene glycol, polypropylene glycol, or copolymers of polyethylene glycol and polypropylene glycol. While not to be constrained by any theoretical mechanism; it is believed that the non-ionic, hydrophilic properties of polyethylene glycol and polypropylene glycol hydrogels provide for both low levels of non-specific binding of analyte to the hydrogel and for good compatibility with the immobilized biomolecules so as to maintain native conformation and bioreactivity thereof. Isocyanate-functional hydrogels advantageously absorb large quantities of liquid quickly and in a relatively uniform manner such that the basic overall shape of the gel material is maintained. Further, the moisture absorbed by these materials is retained in the absorbent material even under an applied pressure. Such isocyanate-functional hydrogels, e.g. polyurethane-based hydrogels are described in U.S. Patent Nos. 3,939,123 (Mathews, et al.) and 4,110,286 (Vandegaer, et al.). These polyurethane-based hydrogels have been extensively used as surface coatings and to form flexible or rigid foams, but not to form a three-dimensional matrix to which attach chemical compounds of interest.

In a preferred embodiment, biochips are made using an isocyanate-functional hydrogel based on a diol or triol of high molecular weight polyethylene oxide, polypropylene oxide, or a copolymer of polyethylene oxide and polypropylene oxide, capped with water-active diisocyanates, and optionally lightly cross-linked with a suitable cross-linker, such that the quantity of active isocyanates present is predictable, for example preferably not greater than about 0.8 meq/g. Generally preferred diisocyanates include aromatic-based diisocyanates, such as toluene diisocyanate or methylene diphenyl-isocyanate, as well as aliphatic diisocyanates, such as isophorone diisocyanate. The polymerization of the prepolymer, which may be preformulated in a water-miscible organic solvent, takes place by the formation of urea linkages which occur simply upon the addition of water. This is a distinct advantage over the hydrogel-based biochips previously known, wherein ultraviolet radiation or similarly severe reaction conditions are necessary to initiate polymerization. Not only is the water-activated polymerization system of the present invention safer, less expensive and easier to control, but it allows for derivatization of the prepolymer with the appropriate biomolecular probe either prior to or simultaneously with polymerization.

In one embodiment described herein, the hydrogel is derivatized prior to polymerization with biomolecules soluble in an organic solvent using a simple two to three-minute reaction between the biomolecules and the isocyanates of the prepolymer. By organic-soluble biomolecules are meant, molecules such as PNAs which have been previously derivatized with amine and which are naturally soluble in organic solvents and those which are modified to be so soluble. In order to prevent premature polymerization of the hydrogel in the present embodiment, the derivatization reaction is carried out in an aprotic water-miscible organic solvent such as, for example, dimethylformamide (DMF), N-methyl-2-pyrrolidinone (NMP), acetone, acetonitrile or the like or mixtures thereof. Thus, prior to swelling of the hydrogel or dispensing of the hydrogel onto the substrate, biomolecular probes are covalently bound to the polyurethane-based prepolymer gel. Following such derivatization, the addition of water initiates polymerization, resulting in biomolecular-derivatized hydrogels, for example, PNA-derivatized hydrogels.

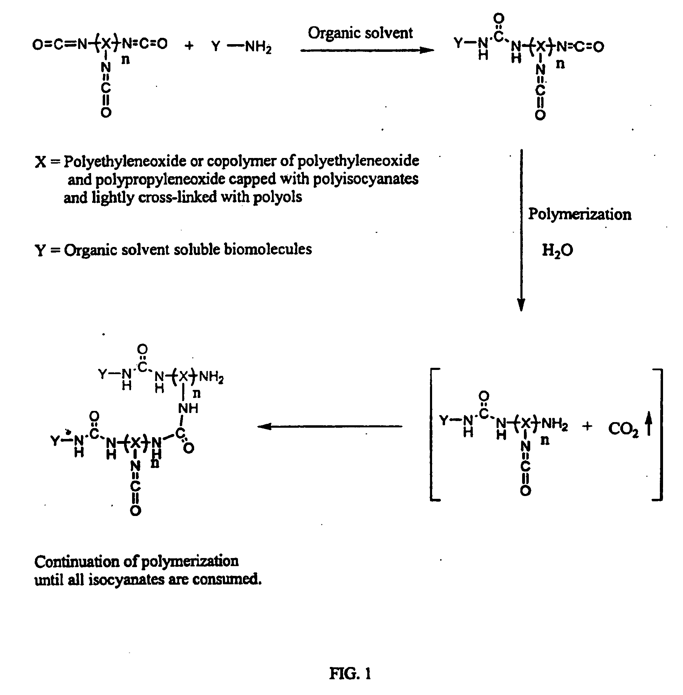

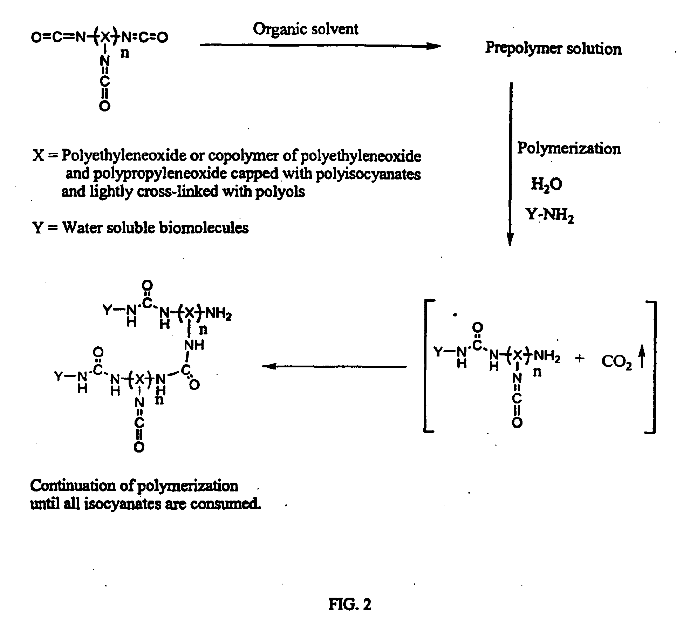

Such use and presence of aprotic solvent in the derivatization of the hydrogel has a number of advantages. First, it helps generate a homogeneous solution of the prepolymer in water. Second, it serves to separate the derivatization step from the polymerization step, whereby controlled yield of biomolecule derivatization to the hydrogel can be achieved. Third, it serves to slow down the generation of carbon dioxide during the polymerization step and effervesce carbon dioxide efficiently by lowering the viscosity of the polymerizing mixture. In the polymerization of the polyurethane-based hydrogels, which in some instances are preferred, carbon dioxide is generated by the reaction of water with the isocyanate groups of the hydrogel prepolymer. This reaction scheme is illustrated, for example in FIGS. 1 and 2, and controlling the generation of carbon dioxide and its escape from the gel may be quite important when a biochip is prepared from such a prepolymer. If polymerization occurs too quickly in a highly viscous mixture, the carbon dioxide generated thereby is not able to escape and becomes trapped within the gel resulting in a discrete foam matrix which may be a problem for continuum of the gel matrix may be important in biochips in order to assure accurate and efficient detection of fluorescence, which is indicative of successful hybridization. The generation of carbon dioxide is controlled by reducing the overall reactivity of water/hydroxide through adding aprotic solvent and by using a relatively low basic pH buffer (pH about 7 to about 8.4), which provides close control of the reaction rate.

A fourth and final advantage to the use of an aprotic solvent to derivatize the hydrogel is the enhancement of the optical transparency of the hydrogel by reducing any precipitation of the prepolymer. It has been discovered that the ratio of aprotic solvent to water should be at least about 0.25 to 1 and preferably higher, e.g. 0.3 - 0.35 to 1, in order to allow desirably slow polymerization of the gel and, therefore, slow generation of $CO_2$, which results in a continuous and transparent gel matrix. Derivatization and polymerization of the hydrogel is generally accomplished in about thirty minutes, which is in stark contrast to the twenty-four to forty-eight hours required for preparation of polyacrylamide-based biochips. Furthermore, the quantity of biomolecules, e.g. PNAs, bound to the prepolymer may easily be adjusted by simply varying the amount of biomolecule added to the reaction (for example, where PNA is the biomolecule to be bound to the gel, from about 10 fmol up to about 1 pmol of FNA may be used for each microdroplet), thereby permitting greater control over the concentration of biomolecule probes within each hydrogel microdroplet.

When the hydrogel is derivatized with PNA then deposited onto the solid substrate, after initiation and before completion of polymerization thereof, such may be accomplished by any convenient method, for example by use of a conventional microspotting machine which preferably deposits gel to form an array of microdroplets. While the gel may inherently attach non-covalently to some substrates, the substrate surface is generally derivatized prior to addition of the hydrogel to achieve maximal attachment of the gel to the substrate. For example, in one preferred embodiment where glass is used as the substrate, the glass is derivatized with amine prior to deposit of the polymerizing hydrogel onto its surface. Thus, the polymerizing hydrogel, derivatized with biomolecular probes such as PNAs or DNAs binds strongly to the substrate when it is deposited onto the derivatized glass substrate, via reaction of active isocyanate groups within the prepolymer with the amines located on the surface of the glass thereby providing covalent attachment of the hydrogel to the substrate.

The biochip substrate may consist of a variety of materials and formats which are conducive to automated handling during use in a binding assay and later detection of molecules binding to the individual biochip cells. For example, an optically transparent substrate, such as glass or polystyrene, will allow for transmission light detection through the cells and is convenient for detection modalities using fluorescence or optical absorption. Due to the high binding capacity of three-dimensional hydrogel cells, reflective optical methods are also possible and allow the use of opaque substrates. The use of rigid substrates allows for precision of alignment during the detection phase of the biochip, but it may not be necessary if proper alignment is incorporated into the cells to facilitate detection. An example would be a flexible format, such as a tape or filament, which could be precisely detected in a scanning fashion similar to the use of magnetic tape. While optical methods and suitable substrates are preferred due to simplicity, other detection methods used in biochemistry could be used, including the detection of radioactive agents.

Advantageously, reactions involved in this system, namely (1) the derivatization of hydrogel prepolymer with the biomolecular probe, (2) the polymerization of hydrogel and (3) the binding of derivatized hydrogel to the substrate surface, involve the formation of strong urea or urethane(carbamate) bonds. These bonds provide mechanical integrity to the microdroplet array and significantly increase the half-life of the biochip, as compared with prior art polyacrylamide-based biochips.

In certain preferred embodiments described hereinafter, the hydrogel droplets, once polymerized on the substrate, are between about 20 $\mu$m and 100 $\mu$m thick; preferably, they are at least about 30 $\mu$m thick and more preferably between about 30 $\mu$m and about 50 $\mu$m thick. The overall larger size of the gel droplets (or cells) permits a significant increase in the quantity of biomolecular probe immobilized therein, thereby increasing the sensitivity of the biochip and facilitating its use.

In alternative embodiments contemplated herein, water-soluble biomolecules, such as DNA or other oligonucleotides, instead of the organic-soluble biomolecules previously described, are bound to the hydrogel. By water-soluble biomolecules are meant molecules such as DNA and RNA that are naturally water-soluble, as well as others which have been modified to be water-soluble. In these embodiments, it is not feasible to first derivatize the hydrogel prepolymer and then initiate polymerization. However, the polyurethane-based hydrogels may advantageously be derivatized and polymerized in a single reaction while the reaction is adequately controlled to provide a derivatized hydrogel having a predictable quantity of water-soluble biomolecular probe attached thereto. In particular, such a hydrogel prepolymer is first dissolved in an organic solvent. The DNA or other water-soluble biomolecule, in aqueous buffer solution, is then added to such prepolymer in a quantity and under appropriate conditions so that the hydrogel both becomes derivatized with the biomolecular probe and polymerizes. As the hydrogel is polymerizing and before the polymerization is complete, it is microspotted onto a suitable substrate, as previously described.

Alternatively, water-soluble biomolecules such as DNA, RNA and many proteins, can be chemically modified render them soluble in aprotic organic solvents, allowing their derivatization to the isocyanate-functional hydrogel prior to polymerization. Modifications may include covalent modifications, such as conjugation with lipids and reversible blocking of ionic groups, as well as non-covalent modifications, such as the use of ion-pairing agents. Likewise, PNAs can be chemically modified to be satisfactorily water-soluble.

**Optimization of Hydrogel Formulation**

The swelling capacity, the polymerization time, and the transparency and strength (i.e., stability) of the ultimate polymer are important characteristics when evaluating a hydrogel for use in a biochip. Optimization of these characteristics provides an optimum hydrogel.

Swelling capacity is an important characteristic of a hydrogel because it reflects the water content. Generally, the higher the water content of the polymerized gel, the faster the diffusion of molecules in and out of the gel. For a biochip, the faster the diffusion of molecules, for example DNA samples, the more efficient the hybridization reactions. The mathematics of molecular diffusion in simple swelling polymers is based on the following semi-empirical equation (Davis B.K. Proc. Natl. Acad. Sci. USA 21:3120, 1974):

$$D_p = D_o \exp [-0.05 + (10^{-6} M) P]$$

wherein,

$D_p$ =    Diffusion coefficient of the specified molecules in the polymer solution
$D_o$ =    Diffusion coefficient of the specified molecules in pure water
$M$ =    Molecular weight of the specified molecules
$P$ =    Percent polymer content

Thus, from this equation it can be seen that the higher the water content, the faster the diffusion of molecules in and out of the polymer. The density and porosity of a hydrogel polymer can be controlled by a variety of parameters including reactant concentrations, the density of reactive groups, and overall reaction kinetics.

Optimizing the time required for polymerization of the hydrogel is particularly important in the processing of a biochip. Ideally, the time required for polymerization should be long enough to permit dispensing of the polymerizing gel onto the surface of the glass substrate with a conventional microspotting machine before polymerization is complete, but short enough that, once dispensed, the hydrogel fully polymerizes shortly thereafter. Based on these requirements, it has been determined that a hydrogel with a polymerization time of about thirty minutes and with a swelling capacity of about. 96% to 97% at equilibrium exhibits good performance. Various experiments were conducted in order to determine the most appropriate formulations and reaction conditions to provide an optimum hydrogel formulation. These experiments included the evaluation of prepolymer-solvent ratios, solvent types and buffer conditions with respect to both gel transparency and polymerization rate. The results are outlined in the following sections.

1. Evaluation of Prepolymers and Polymer-Water Ratios

In initial experiments, it was found that hydrogel prepolymers capped with certain aromatic polyisocyanates polymerized in 2-3 minutes when treated with deionized water, i.e. too quickly for use in biochip production. In contrast, hydrogel prepolymers capped with certain aliphatic polyisocyanates took longer than 35 minutes to complete polymerization under the same conditions, causing them to be selected for further optimization. In order to optimize the prepolymer-water ratio, varying amounts of prepolymer having an active isocyanate content of about 0.4 meq/g were dissolved in water at a pH of about 7, and the respective polymerization times were determined. As shown in Table 1, polymerization time increased as the proportion of prepolymer in the aqueous solution decreased. For example, a 5% prepolymer solution polymerized mainly on the surface, even after 48 hours. In contrast, in unbuffered DI water, the prepolymer solution having 10% prepolymer content polymerized in three hours, and the 20% solution was fully polymerized within about 35 minutes. From these initial experiments, it appeared that a prepolymer solution of at least about 5%, preferably greater than about 10% and more preferably at least about 20% should be used at this pH in the making of biochips; however, it was uncertain how the tentative conclusion might be altered by a change in pH or some other reaction condition.

Table 1

| % prepolymer in aqueous solution | Polymerization time |
|---|---|
| 5% | >48 hours |
| 10% | 3 hours. |
| 20% | 35 minutes |

2. Effect of pH on Polymerization

The next step toward optimization of the hydrogel formulation was to determine the effect of pH on the rate of polymerization. Table 2 summarizes results of these experiments.

Table 2

| % prepolymer in aqueous solution | pH 7.2 | pH 8.4 |
|---|---|---|
| 5% | 90 minutes | 20 minutes |
| 10% | 17 minutes | 3 minutes |

In these experiments, it was found that 50 mM sodium bicarbonate aqueous buffers at pH 7.2 and 8.4 significantly accelerated the polymerization rate of the hydrogel. Thus, for example, when only a 5% prepolymer formulation was used, polymerization time decreased from >48 hours to 90 minutes at pH 7.2 to only 20 minutes at pH 8.4. similarly, the 10% prepolymer solution was fully polymerized within 17 minutes at pH 7.2 and within 3 minutes at pH 8.4. These experiments indicated that, by adjusting the pH of the polymerization reaction solution, a lower prepolymer content, i.e. 5% or 10%, could be used in the hydrogel formulation.
In addition to polymerization time, the swelling properties of the gel should also be considered. While polymerization may occur quickly, the gel is most useful when it has reached the desired water content of at least about 95%, preferably about 96% to 97%. Thus, the swelling characteristics of the different polymer content formulations were also analyzed, and Table 2A compares the swelling profiles of a 5% hydrogel formulation and a 10% hydrogel formulation. Water content is reported in terms of swelling ratio, and swelling ratio is calculated by dividing the total weight of the hydrogel by the weight of the prepolymer.

Table 2A

| Swelling Ratio as a Function of Time and % Prepolymer | | | | |
|---|---|---|---|---|
| | Hours | | | |
| Composition | 1 | 1½ | 3 | 24 |
| 5 % prepolymer | 23 | 25 | 27.5 | 40 |
| 10% prepolymer | 12 | 13 | 15 | 25 |

From these test results, it can be seen that the hydrogel made using a 10% prepolymer solution required about one day to achieve an optimum water content of 96%, which is equal to a swelling ratio of about 25. In addition, the volume of hydrogel, when fully swollen, was about twice its volume just after polymerization began, making the structural integrity of this hydrogel difficult to maintain. In contrast, the hydrogel resulting from a 5% prepolymer solution reached 96% water content in about one hour and did so without loss in structural integrity. Thus, based upon both polymerization rate in this slightly basic solution and swelling profile, the 5% prepolymer solution was selected for still further optimization.

3. Optimizing Transparency of the Hydrogel

A significant disadvantage of most hydrogels made from aqueous solutions is their general opaqueness. To be most useful in a biochip, the gel should be transparent, and in particular, it should be optically transparent so that interference with certain molecular markers or labels, such as fluorescent tags, is minimized. To achieve optical transparency, the hydrogel prepolymer was first dissolved in an aprotic organic solvent, such as acetonitrile, acetone, DMF, NMP or a mixture thereof, and the resulting solution was treated with 50 mM sodium bicarbonate buffer at pH 7.2 or 8.4 to initiate polymerization. $NaHCO_3$ will normally buffer an aqueous solution at a pH of about 8.4; however, the addition of an acid, e.g. HCl, or a base, e.g. NaOH, can be used to slightly alter the buffered pH, e.g. to expand the range to extend from 7.0 to 9.5, although some of the buffer capacity is lost. Using the preferred 5% polymer solution, the ratio of aprotic solvent to aqueous buffer was adjusted to determine an optimum formulation with respect to polymerization time and optical transparency. Table 3 shows the results of testing of five different hydrogel formulations having various ratios of buffer to aprotic solvent, in this case acetonitrile. Each formulation was tested at a buffered pH of 8.4 or 7.2.

Table 3

| Formulation # | Acetonitrile: Buffer | Time to polymerize |
|---|---|---|
| DI water | | |
| control | 0.3 g:0.65 g | 3 hours |
| pH 8.4 buffer | | |
| 1 | 0.1 g: 0.85 g | 45 minutes |
| 2 | 0.2 g: 0.75 g | 42 minutes, |
| 3 | 0.3 g: 0.65 g | 35 minutes |
| 4 | 0.4 g: 0.55 g | 35 minutes |
| 5 | 0.5 g: 0.15 g | 32 minutes |
| pH 7.2 buffer | | |
| 6 | 0.1 g: 0.85 g | 180 minutes |
| 7 | 0.2 g: 0.75 g | 170 minutes |
| 8 | 0.3 g: 0.65 g | 180 minutes |
| 9 | 0.4 g: 0.55 g | 180 minutes |
| 10 | 0.5 g: 0.45 g | 150 minutes |

Each of the tested formulations at pH 8.4 and pH 7.2 polymerized within about 30-45 minutes and 150-180 minutes, respectively, regardless of the relatively large differences in the ratio of acetonitrile to buffer. The higher the pH, the faster the polymerization. It was found that the pH of an earlier prepared sodium bicarbonate buffer increased over time due to the release of $CO_2$ gas from the buffer; thus, when sodium bicarbonate is used as a buffer system, it should be prepared freshly and used the same day. Another buffer system, 50 mM borate buffer, was tested and found to achieve complete polymerization in a time similar to that of freshly-made sodium bicarbonate buffer. A borate buffer is an aqueous solution containing sodium tetraborate and boric acid wherein the proportions can be varied to provide a pH between about 7.0 and about 9.5.

Visual transparencies of each resulting hydrogel varied with respect to the ratio of organic solvent to aqueous buffer. Transparency is believed to be affected by evolution of $CO_2$ and/or by precipitation of the prepolymer. The visual transparency appeared to improve as the ratio of organic solvent to aqueous buffer was increased, which prompted the following additional experiment to be performed.

To measure the effect of visual opacity of the hydrogel upon the optical intensity of fluorescence, the following experiment was performed using three formulations (10%, 20% and 30% aprotic solvents by weight).

Formulation I:

0.2 g Hypol PreMa G-50 was dissolved in 0.22 g acetonitrile and 0.22 g of NMP. The resulting solution is reacted with a DNA solution in 3.8 ml of 50 mM borate buffer pH 8.0 (oligonucleotide concentration, G11, i.e. 5'-CTAAACCTCCAA-3' of 0.75 mg/ml of the buffer). The formulation contained about 10 weight % organic solvents.

Formulation II:

0.2 g Hypol PreMa G-50 was dissolved in 0.44 g acetonitrile and 0.44 g of NMP. The resulting solution is reacted with DNA solution in 3.36 ml of 50 mM borate buffer at pH 8.0 (oligonucleotide concentration, G11, of 0.84 mg/ml of the buffer). The formulation II contained about 20 weight % organic solvents.

Formulation III:

0.2 g Hypol PreMa G-50 was dissolved in 0.67 g of acetonitrile and 0.67 g of NMP. The resulting solution is reacted with DNA solution in 2.89 ml of 50 mM borate buffer at pH 8.0 (oligonucleotide concentration, G11, of 1 mg/ml of the buffer). The formulation III contained about 30 weight % organic solvents.

Formulations I, II and III were microspotted onto glass slides using a conventional microspotting machine, as explained in more detail hereinafter in Example III. After 1 hour curing in a controlled humidifier chamber at a relative humidity of about 95%, the resulting slides were washed with washing buffer and hybridized with fluorescently-labeled target sample for 20 minutes. The intensity of each formulation was measured with a CCD camera. The results are described in Table 3A from which it is clear that the opacity is dependent upon the relative percentage of organic solvent and affects the fluorescence intensity.

Table 3A. Effect of Visual Opacity on the Intensity of Fluorescence

| (organic solvents) | Visual opacity | Fluorescene intensity |
|---|---|---|
| Formulation I (10%) | Opaque | 2044 ($\pm$217) |
| Formulation II (20%) | Moderately opaque | 8223 ($\pm$779) |
| Formulation III (30%) | Transparent | 10209 ($\pm$632) |

As a result of the above testing, it was determined that hydrogel formulations employing at least about 20% and preferably at least about 30% organic solvent by weight of the total solution which is polymerizing and preferably at least about 15% acetonitrile are preferred when optical clarity is desired in the ultimate biochip.

4. Comparison of Aprotic Organic Solvents

The next step in optimization of the hydrogel for the biochips of the present invention, was a comparison of aprotic solvents. Experiments similar to those described in Section II were performed using various aprotic solvents, namely DMF, NMP and acetone. While it was assumed these alternative organic solvents would function similarly to acetonitrile, such was not the case. Results of polymerization studies of hydrogel formulations having 30% DMF, NMP or acetone are summarized in Table 4.

Table 4

| Solvent | pH of buffer | Polymerization time |
|---|---|---|
| DMF | unbuffered DI water | Did not polymerize |
| | 8.4 | Did not polymerize |
| | 9.15 | 20 minutes |
| | 9.5 | 8 minutes |
| | 10 | Did not polymerize |
| NMP | unbuffered DI water | Did not polymerize |
| | 8.4 | Did not polymerize |
| | 9.15 | Did not polymerize |
| | 9.5 | Did not polymerize |
| | 10 | Did not polymerize |
| Acetone | unbuffered DI water | Did not polymerize |
| | 8.4 | 30 minutes |

From these experiments, it was discovered that formulations in DMF required a higher pH to polymerize while, under the same conditions, formulations in NMP did not polymerize at all. Formulations in acetone were most similar to acetonitrile formulations in that they required only slightly basic pH to polymerize. Significantly, the polymerized hydrogels resulting from DMF or acetone formulations were inferior in structural integrity to the polymerized hydrogels produced from acetonitrile formulations. Thus, from these optimization studies, acetonitrile was selected as the preferred aprotic solvent for use in the further development of the optimized biochip; later experiments showed that other aprotic organic solvents such as tetrahydrofuran and dioxane were found to exhibit a similar propensity for achieving complete polymerization as that of acetonitrile and acetone.

Based upon all of the previously described tests, it is generally believed that the pH of the polymerizing solution should be between about 7.0 and about 9.5, preferably between about 7.2 and about 8.6, more preferably between about 7.4 and about 8.4 and most preferably between about 7.6 and about 8.2.

5. <u>Evaluation of Humidity Effects During Polymerization</u>

During or after microspotting, the deposited microdroplets should avoid extensive dehydration or immediate loss of organic solvent. Such loss may result in over-crosslinking during polymerization on the substrate and resultant low diffusability of large molecular weight target samples. This potential problem is reduced by placing the polymerizing biochip into a controlled humidity chamber immediately after microspotting. A controlled humidifier was constructed and maintained at approximately 97 to 98% humidity as determined with a digital sling psychrometer (Model THWD-1, Amprobe Instrument). After microspotting, the biochip was immediately placed within the humidifier chamber. Alternatively, the controlled humidity chamber could be extended to the microspotting process environment.

Table 5 shows the effect of humidity during polymerization on the ultimate intensity of fluorescence of bound biomolecules.

Table 5

| The Effect of Humidity During Polymerization | |
| --- | --- |
| Condition | Fluorescence Intensity |
| Dry | 762 ($\pm$207) |
| Humidity 97-98% | 7161 ($\pm$2454) |

The test shows that there is a nearly 10-fold decrease in intensity when polymerization is carried out under dry conditions. This difference is attributed to overcrosslinking of the polymerized microdroplet when humidified conditions are not employed and consequently low diffusability of target samples. It is thus preferred to expose the microspotted biochip to at least about 90% relative humidity during polymerization.

6. <u>Temperature Control of Hydrogel Polymerization</u>

After the prepolymer mixes with aqueous DNA buffer solution, polymerization begins to take place, resulting in a slow increase in the viscosity of the mixture while it is being applied to the biochip substrate. Although continuous mixing and dispensing processes can be used, it has been found that simple control of reaction kinetics will allow a batch process to be used for preparing and microspotting the polymerizing hydrogel. At room temperature, a significant viscosity change was observed during a five to ten-minute time period, which may affect the size and height of microdroplets during dispensing. This potential problem was essentially eliminated by microspotting in a cold box at 7°C. Lowering the temperature slowed down the polymerization process resulting in more consistent viscosity.

0.2 g of isocyanate-functional hydrogel Hypol PreMa G-50 was dissolved in 0.67 g acetonitrile and 0.67 g N-methyl-2-pyrrolidinone, and the resulting mixture was polymerized by adding 2.89 g of 50 mM borate buffer at pH 8.0. The polymerization was performed at room temperature (~24°C) and at 7°C. The time to completely polymerize was observed and is presented in Table 6.

Table 6

| Polymerization Time at Different Temperatures | |
| --- | --- |
| Temperature | Polymerization Time |
| Room temperature | 50 minutes |
| 7°C | 2 hours |

This testing shows that complete polymerization can be delayed by subjecting the microdroplets to lower temperature, and preferably a temperature of not greater than about 10°C is used.

7. <u>Effect of Thickness on Sensitivity</u>

A solution was prepared by dissolving 0.2 g of Hypol PreMa G-50 in 0.67 g acetonitrile and 0.67 g N-methyl-2-pyrrolidinone. The resulting solution was reacted with a DNA solution in 2.89 ml of 50 mM borate buffer at pH 8.0; the DNA was in the form of the oligonucleotide G11, i.e. 5'-CTAAACCTCCAA-3', at a concentration of 1 mg/mL of the buffer. The overall formulation contained about 30% organic solvents by total weight.

The polymerizing solution of hydrogel was microspotted onto glass slides using a conventional microspotting machine. After curing in the controlled humidifier chamber for 1 hour, resulting slides were washed with washing buffer and then hybridized with a fluorescently-labeled target sample for 20 minutes. The intensity of each formulation was measured with a CCD camera.

Three different heights, 21$\mu$m, 32$\mu$m and 52$\mu$m, of hydrogel microdroplets were produced using the same hydrogel formulation by appropriately adjusting the microspotting mechanism. The heights of microdroplets were measured with a thickness measurement instrument, KLA-Tencor P11. The hydrogel cells were hybridized using standard procedure, and the resulting fluorescence intensities were detected optically and measured. The results are summarized in Table 7.

Table 7

| Effect of Hydrogel Thickness on Fluorescence | |
| --- | --- |
| Thickness ($\mu$m) | Fluorescene Intensity |
| 21 | 2728 ($\pm$480) |
| 32 | 4404 ($\pm$640) |
| 52 | 5616 ($\pm$432) |

The results of the tests show that the thicker the hydrogel cell, the greater the sensitivity.

**<u>Derivatization of the Hydrogel with a Biomolecular Probe</u>**

**Derivatization with a Peptide Nucleic Acid Probe**

**A. Optimization of Reaction Conditions**

Having optimized the hydrogel formulation and its polymerization as indicated, attention was turned to derivatizing the gel with an appropriate biomolecular probe to create a biochip with PNAs, which are considered to be more stable. PNA probes were thus generated for use in derivatizing a preferred hydrogel formulation.

PNAs were first dissolved in acetonitrile, a preferred aprotic solvent, for preparing the hydrogel, but it was discovered that PNAs are only minimally soluble in acetonitrile. A more polar solvent, i.e. NMP, was selected for trial, even though hydrogel formulations using NMP did not polymerize under various of the conditions tested, to see if a solution containing both NMP and acetonitrile might be capable of optimization to provide both adequate solubility for PNAs and favorable polymerization for the hydrogel. Numerous experiments with differing acetonitrile/NMP ratios, determined that an optimum formulation should have a solvent ratio in the range of 3:1 to 1:1 acetonitrile to NMP when those two aprotic solvents were used. An aqueous solution of 50 mM sodium bicarbonate was then used to buffer the polymer/PNA solution at about pH 8.0, creating a formulation which completely polymerized in 20-30 minutes. FIG. 1 illustrates, schematically, the reaction of the hydrogel prepolymer with an organic-soluble biomolecule, such as PNA, and the polymerization of the derivatized prepolymer.

**B. Effect of PNA Derivatization on Polymerization of the Hydrogel**

The next step in providing a PNA biochip, in accordance with this first preferred embodiment, was to evaluate the extent to which the degree of derivatization with PNA affects polymerization of the hydrogel. It was generally considered that the hydrogel formulation in acetonitrile should have an active isocyanate content of at least about 0.1 meq/g and not greater than about 1 meq/g, and that about 0.2-0.8 meq/g would likely be preferred. Using prepolymers within this range, experiments were performed wherein different percentages of the active isocyanates were derivatized with a test compound, benzylamine, for five minutes; the hydrogel used an active isocyanate concentration of about 0.4 meq/g. The derivatized hydrogels were then polymerized by treating with 50 mM sodium bicarbonate buffer at pH 8.0. Derivatization of 10% of the active isocyanates with benzylamine resulted in a polymerized hydrogel having characteristics similar to those of the underivatized, polymerized hydrogel. In contrast, when at least about 20% of the active isocyanates were derivatized with benzylamine, the resulting formulation did not polymerize, indicating that an insufficient quantity of active isocyanates were thereafter available for polymerization. Thus, it is felt that derivatization of about 10% of the active isocyanates of the gel is preferred and that not more than about 15% of the active isocyanates should be so reacted. When 10% of the active isocyanates are derivatized with PNA, it is estimated that about 1 to 2 pmol of PNAs will be bound within a droplet of hydrogel of about 1 nanoliter. control of this parameter is effected by the amount of PNA or other biomolecule added to the prepolymer solution.

**Derivatization with a DNA Probe**

In an alternative embodiment, the hydrogel of the present invention is derivatized with a DNA (or similar oligonucleotide) probe. Unlike PNA however, DNA is not soluble in organic solvent. Therefore, where DNA is used as the biomolecular probe, the derivatization and polymerization steps are not separated but are carried out as a part of a single step. Additionally unlike PNA, DNA does not have an active amine (or other) group available for binding to the active isocyanates of the polymer; thus, DNA is preferably modified, prior to reaction with the prepolymer, to add an appropriate active group, such as by reaction with a diamine or other reagent that would provide an active primary amine, as by using standard amidite chemistry.

As with the previously described embodiment wherein the hydrogel was derivatized with PNA, the organic solvent and aqueous buffer solution amounts and reaction conditions are selected to optimize the hydrogel polymerization with respect to polymerization time, gel transparency and related characteristics. A polymer content of about 4-10% and an organic solvent content of at least about 20% (more preferably at least about 30%) are found to be preferred. It is similarly felt no more than about 20% should be derivatized, preferably not more than about 15%, and more preferably, about 10% or less, of the active isocyanates of the hydrogel, by linking with the DNA probe. Thus, for example, as exemplified below, about 2.5% of the active isocyanates of the hydrogel may be derivatized with the DNA probe, with polymerization preferably being accomplished using a basic buffered aqueous solution, at about pH 7.2 to about 8.6, and more preferably about 7.6 to about 8.2.

To prepare this derivatized hydrogel, the prepolymer is first dissolved in an organic solvent, for example, acetonitrile, and the derivatized DNA (or other water-soluble oligonucleotide) probe is prepared in aqueous buffer. The DNA solution is then added to the prepolymer solution with thorough mixing, resulting in both derivatization of the gel and initiation of polymerization. This general method of dissolving a polyurethane-based.hydrogel in organic solvent and adding thereto a water-soluble biomolecular probe in aqueous solution is applicable to numerous water-soluble probes in addition to oligonucleotides. FIG. 2 illustrates, schematically, the simultaneous reaction of a water-soluble biomolecule, such as DNA, with the hydrogel prepolymer and polymerization of the hydrogel.

**Binding of Hydrogel to the Surface of the Substrate**

The final step is that of dispensing the derivatized hydrogel prepolymer formulation onto a suitable substrate under appropriate conditions to permit binding of the hydrogel to the substrate surface. When the substrate selected is transparent, in order to reduce interference with signal detection when the biochip is scanned by transmitted light, a preferred substrate is glass, the surface of which is first derivatized with amine, in order to provide active sites for covalent binding of the polymerizing hydrogel. Just prior to dispensing the derivatized hydrogel onto the derivatized glass substrate, polymerization is initiated either by addition of aqueous buffer to the derivatized prepolymer (in the case of derivatization with an organic-solvent-soluble biomolecule) or by addition of aqueous buffer which includes a water-soluble biomolecule. The-polymerizing hydrogel is then microspotted onto the glass substrate to form an array of microdroplets most preferably having a thickness of about 30 to 50 $\mu$m.

A 50 mM sodium bicarbonate aqueous buffer solution, pH from about 7.2 to about 8.4, may be used to initiate polymerization of the hydrogel, and the hydrogel formulation binds tightly to the surface of such derivatized glass within about 1 to 10 minutes from the initiation of polymerization. When PNA or another organic-soluble probe is used, the derivatized prepolymer need not be used immediately following derivatization, but it may be stored under proper conditions prior to initiation of polymerization by addition of aqueous buffer. In contrast, when the prepolymer is derivatized with a water-soluble probe, polymerization and derivatization begin upon addition of the probe to the prepolymer solution so the polymerizing hydrogel should be dispensed shortly thereafter.

Advantageously, these isocyanate-functional hydrogels polymerize quickly into very stable microdroplets, and such are preferably dispensed onto a substrate to form microdroplets of at least about 20 $\mu$m in height, more preferably at least about 30 $\mu$m in height, and most preferably about 30 $\mu$m to about 50 $\mu$m in height. Consistency between hydrogel droplets is obtained by dispensing the polymerizing gel using a microspotting or similar automated machine, as providing such thick gel droplets, or gel cells, can dramatically increase the sensitivity of the biochip.

In order to prevent overcrosslinking of the polymerizing gel, which can result in an increase in the polymer concentration as water is evaporated from the gel and which may also cause wrinkling in the surface of the gel droplet, the biochip is preferably cured in a humid environment, such as a humidity chamber, and thereafter washed with deionized water.

The viscosity and sheathing properties of the polymerizing hydrogel against the glass substrate may be important to provide a reasonable and consistent shape of droplets throughout the array. In an ideal situation, the viscosity of the polymerizing hydrogel increases slowly and in a linear fashion. Unfortunately, however, the viscosity of hydrogels tend to increase exponentially during polymerization. To facilitate a more linear expansion of the hydrogel during polymerization, its viscosity may be modified using thickening agents, such as polyethylene glycol of various molecular weights. The viscosity of the hydrogel during polymerization may be measured, for example with a viscometer, following addition of the thickening agent. From experimentation, it was determined that addition of polyethylene glycol having a molecular weight of about 1,000 or higher, e.g. as high as the molecular weight of the prepolymer, could significantly improve the formulation's viscosity and sheathing properties. Thus, in certain preferred embodiments, polyethylene glycol or a similar thickening agent is added to the hydrogel during polymerization thereof to control the rate of polymerization.

### Example I: Preparation of a PNA Biochip and Test

A solution of 1 mg PNA (0.3 $\mu$m) having the sequence $NH_2$-CATTGCTCAAAC-$CO_2$H was prepared in 0.1 g of NMP. Next, a solution of 0.05 g of Hypol PreMa G-50, a polyurethane prepolymer having an active isocyanate content of about 0.4 meq/g, was prepared in 0.2 g acetonitrile and added to the PNA-NMP solution. The resulting solution was treated with 0.65 g of a 50 mM $NaHCO_3$ solution at about pH 8.0. After thorough mixing, two droplets of the resulting solution were manually spotted on a silanated glass slide using a capillary microtube. The droplets polymerized on the surface of the glass in about 15 minutes. As a negative control, two hydrogel droplets containing no PNA were spotted next to the PNA-containing hydrogel droplets.

The glass slide, having the hydrogel droplets thereon, was submersed into washing buffer (5 mM sodium phosphate buffer with 0.05% SDS at pH 7.0) for 30 minutes to remove organic solvents and block the remaining active sites to prevent non-specific binding of test DNA. Next, the slide was treated with 1 mg of a complementary fluorescence-labeled DNA, 3'-TAGTAACGAGTTTGCC-5'-Fluorescein, in hybridization buffer containing 600 $\mu$L of 10 mM sodium phosphate buffer with 0.1% SDS at pH 7.0 at room temperature, for 1 hour. Non-specifically bound DNA was removed by washing for two hours in washing buffer. The resulting slide was observed with a hand-held fluorescence detector (Model UVGL-25, UVP). The test DNA diffused into the hydrogel microdroplets and hybridized to the complementary PNA capture probe sequence, providing a strong fluorescent signal, while the test DNA had been washed away from the negative control droplets which gave no signal. The test demonstrates the usefulness of such hydrogel biochips.

### Example II: Preparation of a DNA Biochip and Test

A solution of 0.025 g of Hypol PreMa G-50 was prepared in 0.15 g acetonitrile. Next, a solution of 1 mg DNA (0.3 $\mu$m), having hexaneamine at its 5' end and having the sequence $NH_2(CH_2)_6$-CATTGCTCAAAC-3', in 0.32 g of a 50 mM $NaHCO_3$ aqueous buffer at pH 8.0 was prepared. The DNA solution was added to the prepolymer solution and thoroughly mixed. Droplets of the resulting solution were manually spotted on a silanated glass slide using a capillary microtube. As a negative control, hydrogel droplets containing no DNA were spotted next to the DNA-containing hydrogel droplets.

The glass slide, having the hydrogel droplets thereon, was submersed into washing buffer (10 mM sodium phosphate buffer with 0.5 M NaCl and 0.1% SDS at pH 7.0) for 30 minutes to remove organic solvents and block the remaining active sites to prevent non-specific binding of test DNA. Next, the slide was treated with 1 mg of a complementary fluorescence-labeled DNA, 3'-TAGTAACGAGTTTGCC-5'-Fluorescein, in 600 $\mu$L hybridization buffer (10 mM sodium phosphate buffer with 0.5 M NaCl and 0.1% SDS at pH 7.0) at room temperature, for 1 hour. Non-specifically bound DNA was removed by washing for two hours in washing buffer. The slide was observed with a hand-held fluorescence detector (Model UVGL-25, UVP). The complementary, test DNA diffused into the hydrogel microdroplet and hybridized to the gel-bound DNA probe sequence resulting in a strong fluorescent signal, but it was washed away from the negative control droplet, demonstrating the reliability and usefulness of the present hydrogel biochips in DNA hybridization assays.

### Example III. Preparation of an Array DNA Biochip and Test in Human β-globin Gene Sequence Detection

A DNA biochip was prepared as follows:

    1. The following two reactant solutions were prepared:

        Solution A = 0.1 g Hypol Pre-Ma G-50 in 0.33 g acetonitrile and 0.33 g NMP (Weight ratio of 4.5:15:15)

Solution B = 1 mg of oligonucleotide in 1 ml of 50 mM borate buffer at pH 8.0

2. Solution A (34.5 parts) was mixed with Solution B (65.5 parts), and the resultant solution microspotted onto a glass slide. Microspotting was performed with an open configuration pin, CT MicroPipets DP-120 μm, supplied by Conception Technologies.

3. The microspotted slides were placed into a controlled humidifier chamber for one hour and then washed with a washing buffer for 10 minutes, completing the preparation of the biochips.

Testing of such a biochip is performed by hybridization with a target sample carrying a fluorescent tag or the like at different concentrations in a hybridization buffer system for 20 minutes to 2 hours, proportional to the molecular weight of the target. Any non-specifically bound target is washed away with the hybridization buffer, and the biochip is then scanned to detect the bound target by optical fluorescence.

To validate the performance of these biochips which carry DNA probes, the following twenty 12-mer oligonucleotides, derivatized with primary amine at the respective 5' end using standard amidite chemistry, were immobilized on separate hydrogel cells as a part of a biochip made in this manner:

|     |                   |
|-----|-------------------|
| G1  | 5'-CCTAAGTTCATC-3' |
| G2  | 5'-TATCTCTTATAG-3' |
| G3  | 5'-CTATCGTACTGA-3' |
| G4  | 5'-TTCCTTCACGAG-3' |
| G5  | 5'-ATTATTCCACGG-3' |
| G6  | 5'-ATCTCCGAACTA-3' |
| G7  | 5'-CCTTATTATGCA-3' |
| G8  | 5'-ACGCTTCCTCAG-3' |
| G9  | 5'-GACTTCCATCGG-3' |
| G10 | 5'-CGTACCTTGTAA-3' |
| G11 | 5'-CTAAACCTCCAA-3' |
| G12 | 5'-CTAGCTATCTGG-3' |
| G13 | 5'-TAATTCCATTGC-3' |
| G14 | 5'-ATTCCGATCCAG-3' |
| G15 | 5'-TTAGTTATTCGA-3' |
| G16 | 5'-AAGTTCATCTCC-3' |
| G17 | 5'-TTCATCTCCGAA-3' |
| G18 | 5'-CCGAACTAAACC-3' |
| G19 | 5'-AACTAAACCTCC-3' |
| G20 | 5'-CTAAACGTCCAA-3' |
| G21 | Blank hydrogel    |

A target 30-mer DNA sample from the sequence of the human β-globin gene was synthesized and labeled with a tagging molecule, i.e. fluorescein, at its 5' end using standard amidite chemistry. The sequence of this target sample is the following:

5'-(Fluorescein)-TTGGAGGTTTAGTTCGGAGATGAACTTAGG-3'

The sequences of G1, G6, G11, G16, G17, G18 and G19 are fully complementary to different regions of the target sample. The sequence of G20 has an internal one-base pair mismatch from that of G11. The results of the testing are set forth in Table 8 which follows:

Table 8

| The Intensity of Fluorescence Depending on Sequences | | | | | | | |
|-----------------|------|------|------|------|------|------|------|------|
| Oligonucleotide | G1   | G6   | G11  | G16  | G17  | G18  | G19  | G20  |
| Intensity       | 1528 | 2713 | 5630 | 650  | 841  | 2098 | 6066 | 2181 |
| Standard deviation | 77 | 151  | 238  | 164  | 127  | 354  | 638  | 225  |

As seen in Table 8, the hybridization discrimination of perfect match (G11) and one-base pair mismatch (G20) was excellent (Fluorescence intensity of 5630 vs 2181). The non-related oligonucleotides of G2, G3, G4, G5, G7, G8, G9, G10, G12, G13, G14 and G15, as well as the blank hydrogel cell, demonstrated intensity just above background showing minimum non-specific binding to the hydrogel.

**Claims**

1.  A biochip comprising:

    (a) a solid substrate having a top surface;
    (b) a plurality of hydrogel cells, each having an individual thickness of at least 20 $\mu$m, attached to the top surface of the substrate; and
    (c) a biomolecular probe covalently bound to and

    within at least one of the hydrogel cells, **characterized in that** said cells are formed of an isocyanate-functional polymer and are optically transparent and **in that** different probes are bound to and within various of said cells.

2.  The biochip of claim 1 further **characterized in that** each hydrogel cell comprises a polymer with urethane and urea linkages.

3.  The biochip of claim 1 or 2 further **characterized in that** the hydrogel polymer comprises polyethylene glycol, polypropylene glycol or a copolymer thereof.

4.  The biochip of any one of claims 1, 2 or 3 wherein the substrate is transparent and has reactive molecules on its top surface which covalently bind the hydrogel to the substrate as a result of the isocyanate functionality.

5.  The biochip of any one of claims 1-4 wherein said cells are between 20 $\mu$m thick and 100 $\mu$m thick.

6.  The biochip of any one of claims 1-5 wherein the biomolecular probe comprises DNA, RNA or PNA.

7.  A method of preparing a hydrogel biochip having a biomolecule immobilized thereon, the method comprising the steps of:

    (a) providing a solid substrate having a top surface;
    (b) providing a solution of a prepolymer in an aprotic water-miscible organic solvent;
    (c) providing a solution of a biomolecule;
    (d) covalently binding the biomolecule to the prepolymer; and
    (e) initiating polymerization of the hydrogel prepolymer in aqueous solution at a pH from 7.0 to 9.5, **characterized in that** said prepolymer is an isocyanate-functional hydrogel prepolymer and **in that** said polymerization is controlled so that the resulting hydrogel has adequate pore size to allow biomolecule diffusion and is optically transparent in its fully polymerized state.

8.  The method according to claim 7 wherein the aprotic water-miscible organic solvent is selected from the group consisting of N-methyl-2-pyrrolidinone (NMP), dimethylformamide (DMF), tetrahydrofuran, dioxane, acetone, acetonitrile and mixtures thereof.

9.  The method according to claim 7 or 8 wherein the aprotic water-miscible organic solvent is acetone or acetonitrile.

10. The method according to any one of claims 7-9 further **characterized in that** the hydrogel comprises a polymer with urethane-urea leakages formed from isocyanate crosslinking and **in that** the biomolecule is covalently bound to the prepolymer through a bond resulting from isocyanate reactivity of the substrate.

11. The method according to claim 10 wherein said prepolymer reacts with a primary amine of the biomolecule, which is further **characterized in that** the substrate has active amines an its top surface which also react with isocyanate groups to covalently bind the hydrogel to the substrate.

12. The method according to any one of claims 7-11 further **characterized in that** the hydrogel prepolymer is provided as a solution having active isocyanates of between about 0.1 meq/g and about 1 meq/g and **in that** said prepolymer comprises polyethylene oxide, polypropylene oxide or a copolymer of polyethylene oxide and polypropylene oxide capped with diisocyanates, which prepolymer may be optionally lightly cross-linked.

13. The method according to any one of claims 7-12 further **characterized in that** the steps of covalently binding the biomolecule to the hydrogel prepolymer and initiating polymerization of the hydrogel prepolymer are performed simultaneously by mixing the prepolymer solution with an aqueous solution of said biomolecule.

14. The method according to any one of claims 7-13 which is further **characterized in that** the pH and an amount of aprotic solvent present in the hydrogel solution are used to control carbon dioxide evolution and avoid precipitation of isocyanate-functional prepolymer so as to minimize opacity of the resulting hydrogel, and **in that** polymerization is carried out under at least 90% relative humidity.

15. The method of any one of claims 7-12 further **characterized in that** the biomolecule is dissolved in an aprotic organic solvent and polymerization of the hydrogel prepolymer is initiated by adding a buffered aqueous solution thereto, with the ratio of organic solvent to water being greater than 0.25 to 1 at the time the hydrogel is polymerizing, wherein the method further includes the step of microspotting the polymerizing hydrogel solution onto the top surface of the substrate, resulting in attachment of the hydrogel and immobilized biomolecule to the substrate.

16. The method according to any one of claims 7-12 wherein the active isocyanate content of the hydrogel prepolymer is between 0.2 and 0.8 meq/g, wherein 10% or less of the reactive isocyanates in the prepolymer react to immobilize the biomolecular probes, with other reactive isocyanates covalently binding to the substrate and forming a cross-linked hydrogel.

17. A hybridization assay comprising:

(a) providing a biochip which comprises a substrate having at least two hydrogel cells bound thereto, each cell having a thickness of at least 20 $\mu$m and comprising a polymer formed from an isocyanate-functional hydrogel prepolymer prepared from polyethylene glycol, polypropylene glycol or a copolymer thereof, wherein at least one hydrogel cell further includes a biomolecular probe covalently bound thereto;
(b) contacting the biochip with an analyte solution contacting a target biomolecule, under hybridization conditions;
(c) washing the hydrogel biochip under conditions that remove non-specifically bound and unbound target biomolecule; and
(d) detecting the bound target biomolecule.

**Patentansprüche**

1. Biochip, umfassend

(a) ein festes Substrat mit einer oberen Oberfläche;
(b) eine Vielzahl Hydrogelzellen, jede mit einer individuellen Dicke von mindestens 20 $\mu$m, die an die obere Oberfläche des Substrats angeheftet sind; und
(c) eine biomolekulare Sonde, die kovalent an und in mindestens eine der Hydrogelzellen gebunden ist,

**dadurch gekennzeichnet, dass** diese Zellen aus einem Isocyanat-funktionalen Polymer gebildet werden und optisch transparent sind, und **dadurch dass** verschiedene Sonden an und in verschiedene dieser Zellen gebunden sind.

2. Biochip gemäß Anspruch 1, ferner **dadurch gekennzeichnet, dass** jede Hydrogelzelle ein Polymer mit Urethan- und Harnstoffbindungen umfasst.

3. Biochip gemäß Anspruch 1 oder 2, ferner **dadurch gekennzeichnet, dass** das Hydrogelpolymer Polyethylenglykol, Polypropylenglykol oder ein Copolymer davon umfasst.

4. Biochip gemäß irgendeinem der Ansprüche 1, 2 oder 3, worin das Substrat transparent ist und reaktive Moleküle auf seiner oberen Oberfläche hat, die das Hydrogel kovalent an das Substrat binden, infolge der Isocyanatfunktionalität.

**5.** Biochip gemäß irgendeinem der Ansprüche 1 bis 4, worin die Zellen zwischen 20 $\mu$m dick und 100 $\mu$m dick sind.

**6.** Biochip gemäß irgendeinem der Ansprüche 1 bis 5, worin die biomolekulare Sonde DNA, RNA oder PNA umfasst.

**7.** Verfahren zum Herstellen eines Hydrogel-Biochips mit einem darauf immobilisierten Biomolekül, wobei das Verfahren die Schritte umfasst:

(a) Bereitstellen eines festen Substrats mit einer oberen Oberfläche;
(b) Bereitstellen einer Lösung eines Präpolymers in einem aprotischen wassermischbaren organischen Lösungsmittel;
(c) Bereitstellen einer Lösung eines Biomoleküls;
(d) kovalentes Binden des Biomoleküls an das Präpolymer; und
(e) Initiieren der Polymerisierung des Hydrogel-Präpolymers in wässriger Lösung bei einem pH von 7,0 bis 9,5,

**dadurch gekennzeichnet, dass** das Präpolymer ein Isocyanat-funktionales Hydrogel-Präpolymer ist, und dass die Polymerisierung so kontrolliert wird, dass das entstehende Hydrogel eine adäquate Porengröße hat, um die Biomolekül-Diffusion zu ermöglichen und in seinem vollständig polymerisierten Zustand optisch transparent ist.

**8.** Verfahren gemäß Anspruch 7, worin das aprotische wassermischbare organische Lösungsmittel ausgewählt wird aus der Gruppe, bestehend aus N-Methyl-2-pyrrolidon (NMP), Dimethylformamid (DMF), Tetrahydrofuran, Dioxan, Aceton, Acetonitril und Gemischen daraus.

**9.** Verfahren gemäß Anspruch 7 oder 8, worin das aprotische wassermischbare organische Lösungsmittel Aceton oder Acetonitril ist.

**10.** Verfahren gemäß irgendeinem der Ansprüche 7 bis 9, weiter **dadurch gekennzeichnet, dass** das Hydrogel ein Polymer mit Urethan-Harnstoff-Durchlassen umfasst, die durch Isocyanatvernetzung gebildet werden, und **dadurch dass** das Biomolekül kovalent an das Präpolymer durch eine Bindung gebunden wird, die aus der Isocyanatreaktivität des Substrats entsteht.

**11.** Verfahren gemäß Anspruch 10, worin das Präpolymer mit einem primären Amin des Biomoleküls reagiert, welches weiter **dadurch gekennzeichnet ist, dass** das Substrat aktive Amine an seiner oberen Oberfläche hat, die ebenfalls mit Isocyanatgruppen reagieren, um das Hydrogel kovalent an das Substrat zu binden.

**12.** Verfahren gemäß irgendeinem der Ansprüche 7 bis 11, ferner **dadurch gekennzeichnet, dass** das Hydrogel-Präpolymer als eine Lösung mit aktiven Isocyanaten von zwischen ungefähr 0,1 mÄq/g und ungefähr 1 mÄq/g bereitgestellt wird, und **dadurch, dass** dieses Präpolymer Polyethylenoxid, Polypropylenoxid oder ein Copolymer aus Polyethylenoxid und Polypropylenoxid umfasst, welches mit Diisocyanaten verkappt ist, wobei das Präpolymer gegebenenfalls leicht vernetzt sein kann.

**13.** Verfahren gemäß irgendeinem der Ansprüche 7 bis 12, weiter **dadurch gekennzeichnet, dass** die Schritte des kovalenten Bindens des Biomoleküls an das Hydrogel-Präpolymer und des Initiierens der Polymerisierung des Hydrogel-Präpolymers gleichzeitig durch Mischen der Präpolymerlösung mit einer wässrigen Lösung dieses Biomoleküls durchgeführt werden.

**14.** Verfahren gemäß irgendeinem der Ansprüche 7 bis 13, welches weiter **dadurch gekennzeichnet ist, dass** der pH-Wert und eine Menge des aprotischen Lösungsmittels, die in der Hydrogellösung vorhanden sind, verwendet werden, um die Entstehung von Kohlendioxid zu kontrollieren und die Präzipitierung von Isocyanat-funktionalem Präpolymer zu verhindern, um die Opazität des erhaltenen Hydrogels zu minimieren, **dadurch, dass** die Polymerisierung bei mindestens 90 % relativer Feuchtigkeit durchgeführt wird.

**15.** Verfahren gemäß irgendeinem der Ansprüche 7 bis 12, weiter **dadurch gekennzeichnet, dass** das Biomolekül in einem aprotischen organischen Lösungsmittel gelöst ist, und die Polymerisierung des Hydrogel-Präpolymers durch Zugeben einer gepufferten wässrigen Lösung hierzu initiiert wird, bei einem Verhältnis des organischen Lösungsmittels zu Wasser, das größer als 0,25 bis 1 ist, zum Zeitpunkt, bei dem das Hydrogel polymerisiert, worin das Verfahren weiter den Schritt des Mikrospottings der polymerisierten Hydrogellösung auf die obere Oberfläche des Substrats einschließt, was zur Haftung des Hydrogels und des immobilisierten Biomoleküls an das Substrats führt.

**16.** Verfahren gemäß irgendeinem der Ansprüche 7 bis 12, worin der aktive Isocyanatgehalt des Hydrogel-Präpolymers zwischen 0,2 und 0,8 mÄq/g liegt, worin 10 % oder weniger der reaktiven Isocyanate in dem Präpolymer reagieren, um die biomolekularen Sonden zu immobilisieren, wobei andere reaktive Isocyanate kovalent an das Substrat binden und ein vernetztes Hydrogel bilden.

**17.** Hybridisierungsassay, umfassend

(a) Bereitstellen eines Biochips, welcher ein Substrat umfasst, das mindestens zwei Hydrogelzellen daran gebunden hat, wobei jede Zelle eine Dicke von mindestens 20 $\mu$m hat und ein Polymer umfasst, das aus einem Isocyanat-funktionalen Hydrogel-Präpolymer gebildet wird, was aus Polyethylenglykol, Polypropylenglykol oder einem Copolymer davon gebildet wird, worin mindestens eine Hydrogelzelle außerdem eine biomolekulare Sonde einschließt, die kovalent daran gebunden ist;
(b) Kontaktieren des Biochips mit einer Analyt-Lösung, die das Ziel-Biomolekül unter Hybridisierungsbedingungen kontaktiert;
(c) Waschen des Hydrogel-Biochips unter Bedingungen, die unspezifisch gebundene und nicht-gebundene Ziel-Biomoleküle entfernen; und
(d) Nachweisen des gebundenen Ziel-Biomoleküls.

## Revendications

**1.** Biopuce comprenant :

(a) un substrat solide ayant une surface supérieure ;
(b) une pluralité de cellules d'hydrogel, chacune ayant une épaisseur individuelle d'au moins 20 $\mu$m, fixées à la surface supérieure du substrat ; et
(c) une sonde biomoléculaire liée par covalence à, et dans, au moins une des cellules d'hydrogel,

**caractérisée en ce que** lesdites cellules sont formées d'un polymère à fonctionnalité isocyanate et sont optiquement transparentes et **en ce que** des sondes différentes sont liées à, et dans, diverses telles cellules.

**2.** Biopuce suivant la revendication 1, **caractérisée en outre en ce que** chaque cellule d'hydrogel comprend un polymère avec des liaisons uréthanne et urée.

**3.** Biopuce suivant la revendication 1 ou 2, **caractérisée en outre en ce que** le polymère d'hydrogel comprend le polyéthylèneglycol, le polypropylèneglycol ou un de leurs copolymères.

**4.** Biopuce suivant l'une quelconque des revendications 1, 2 et 3, dans laquelle le substrat est transparent et porte des molécules réactives sur sa surface supérieure qui lient par covalence l'hydrogel au substrat en résultat de la fonctionnalité isocyanate.

**5.** Biopuce suivant l'une quelconque des revendications 1 à 4, dans laquelle lesdites cellules ont une épaisseur comprise entre 20 $\mu$m et 100 $\mu$m.

**6.** Biopuce suivant l'une quelconque des revendications 1 à 5, dans laquelle la sonde biomoléculaire comprend un ADN, un ARN ou un PNA.

**7.** Procédé pour la préparation d'une biopuce d'hydrogel comprenant une biomolécule immobilisée sur celle-ci, procédé comprenant les étapes consistant à :

(a) fournir un substrat solide ayant une surface supérieure ;
(b) fournir une solution d'un prépolymère dans un solvant organique aprotique miscible à l'eau ;
(c) fournir une solution d'une biomolécule ;
(d) lier par covalence la biomolécule au prépolymère ; et
(e) déclencher la polymérisation du prépolymère d'hydrogel en solution aqueuse à un pH de 7,0 à 9,5, **caractérisé en ce que** ledit prépolymère est un prépolymère d'hydrogel à fonctionnalité isocyanate et **en ce que** ladite polymérisation est régulée de telle sorte que l'hydrogel résultant ait un diamètre des pores adéquat pour permettre la diffusion de biomolécules et soit optiquement transparent à l'état totalement polymérisé.

8. Procédé suivant la revendication 7, dans lequel le solvant organique aprotique miscible à l'eau est choisi dans le groupe consistant en N-méthyl-2-pyrrolidinone (NMP), diméthylformamide (DMF), tétrahydrofuranne, dioxanne, acétone, acétonitrile et leurs mélanges.

9. Procédé suivant la revendication 7 ou 8, dans lequel le solvant organique aprotique miscible à l'eau est l'acétone ou l'acétonitrile.

10. Procédé suivant l'une quelconque des revendications 7 à 9, **caractérisé en outre en ce que** l'hydrogel comprend un polymère avec des liaisons uréthanne-urée formées par réticulation d'isocyanate et **en ce que** la biomolécule est liée par covalence au prépolymère par une liaison résultant de la réactivité du substrat avec la fonctionnalité isocyanate.

11. Procédé suivant la revendication 10, dans lequel ledit prépolymère réagit avec une amine primaire de la biomolécule, **caractérisé en outre en ce que** le substrat possède des amines actives à sa surface supérieure qui réagissent également avec les groupes isocyanate pour lier par covalence l'hydrogel au substrat.

12. Procédé suivant l'une quelconque des revendications 7 à 11, **caractérisé en outre en ce que** le prépolymère d'hydrogel est fourni sous forme d'une solution comprenant des isocyanates actifs à une concentration d'environ 0,1 meq/g à environ 1 meq/g et **en ce que** ledit prépolymère comprend le poly(oxyde d'éthylène), le poly(oxyde de propylène) ou un copolymère de poly(oxyde d'éthylène) et de poly(oxyde de propylène) coiffé avec des diisocyanates, prépolymère qui peut être facultativement légèrement réticulé.

13. Procédé suivant l'une quelconque des revendications 7 à 12, **caractérisé en outre en ce que** les étapes de liaison par covalence de la biomolécule au prépolymère d'hydrogel et de déclenchement de la polymérisation du prépolymère d'hydrogel sont mises en oeuvre simultanément en mélangeant la solution de prépolymère à une solution aqueuse de ladite biomolécule.

14. Procédé suivant l'une quelconque des revendications 7 à 13, **caractérisé en outre en ce que** le pH et une quantité de solvant aprotique présent dans la solution d'hydrogel sont utilisés pour ajuster le dégagement de dioxyde de carbone et éviter la précipitation du prépolymère à fonctionnalité isocyanate afin de réduire au minimum l'opacité de l'hydrogel résultant, et **en ce que** la polymérisation est effectuée à une humidité relative d'au moins 90 %.

15. Procédé suivant l'une quelconque des revendications 7 à 12, **caractérisé en outre en ce que** la biomolécule est dissoute dans un solvant organique aprotique et la polymérisation du prépolymère d'hydrogel est déclenchée en y ajoutant une solution aqueuse tamponnée, le rapport du solvant organique à l'eau étant de plus de 0,25 à 1 lors de la polymérisation de l'hydrogel, le procédé comprenant en outre l'étape de dépôt sous forme de microtaches de la solution d'hydrogel pour polymérisation sur la surface supérieure du substrat, avec pour résultat la fixation de l'hydrogel et de la biomolécule immobilisée au substrat.

16. Procédé suivant l'une quelconque des revendications 7 à 12, dans lequel la teneur en isocyanate actif du prépolymère d'hydrogel est comprise entre 0,2 et 0,8 meq/g, une proportion égale ou inférieure à 10 % des isocyanates réactifs dans le prépolymère réagissant pour immobiliser les sondes biomoléculaires, d'autres isocyanates réactifs se liant par covalence au substrat et formant un hydrogel réticulé.

17. Analyse d'hybridation, comprenant les étapes consistant à :

(a) fournir une biopuce qui comprend un substrat auquel sont liées au moins deux cellules d'hydrogel, chaque cellule ayant une épaisseur d'au moins 20 μm et un polymère formé à partir d'un prépolymère d'hydrogel à fonctionnalité isocyanate préparé à partir de polyéthylèneglycol, de polypropylèneglycol ou d'un de leurs copolymères, au moins une cellule d'hydrogel comprenant en outre une sonde biomoléculaire liée par covalence à celle-ci ;
(b) à mettre en contact la biopuce avec une solution d'analyte entrant en contact avec une biomolécule cible, dans des conditions d'hybridation ;
(c) laver la biopuce d'hydrogel dans des conditions qui éliminent la biomolécule cible liée non spécifiquement et non liée ; et
(d) détecter la biomolécule cible liée.

X = Polyethyleneoxide or copolymer of polyethyleneoxide
and polypropyleneoxide capped with polyisocyanates
and lightly cross-linked with polyols

Y = Organic solvent soluble biomolecules

Continuation of polymerization
until all isocyanates are consumed.

FIG. 1

X = Polyethyleneoxide or copolymer of polyethyleneoxide
and polypropyleneoxide capped with polyisocyanates
and lightly cross-linked with polyols

Y = Water soluble biomolecules

Continuation of polymerization
until all isocyanates are consumed.

FIG. 2